# EUROPEAN PATENT SPECIFICATION

(11) **EP 1 429 748 B1**
(45) Date of publication and mention of the grant of the patent: **20.07.2005**
(21) Application number: 02758020.8
(22) Date of filing: 12.09.2002
(51) Int. Cl.: A61K 9/48, A61K 47/26, A61K 31/40, A61K 38/55

(54) **SOLID COMPOSITIONS COMPRISING RAMIPRIL**
FESTE ZUBEREITUNGEN MIT RAMIPRIL
COMPOSITIONS SOLIDES COMPRENANT DU RAMIPRIL

(30) Priority: 28.09.2001 CA 2357982
(43) Date of publication of application: 23.06.2004
(73) Proprietor: Sherman, Bernard Charles, Toronto, Ontario M2L 2K1 (CA)
(72) Inventor: Sherman, Bernard Charles, Toronto, Ontario M2L 2K1 (CA)
(74) Representative: Howard, Paul Nicholas
(86) International application number: PCT/CA2002/001379
(87) International publication number: WO 2003/028707

(56) References cited:
- EP-A- 0 317 878
- WO-A-00/34314
- WO-A-02/11709
- WO-A-96/07400
- DE-A- 4 420 102
- US-A- 5 562 921
- FIEDLER, HERBERT P: "Lexikon der Hilfsstoffe für Pharmazie, Kosmetik und angrenzende Gebiete" 2002 , EDITIO CANTOR VERLAG , AULENDORF XP002222074 page 996, column 2, last paragraph

## Description

### BACKGROUND OF THE INVENTION

Ramipril is a medicinal compound that inhibits angiotensin-converting enzyme ("ACE") and is thus useful as an antihypertensive agent. It is disdosed in U.S. patent 5,061,722 and specifically claimed by daim 2 of that patent.

Capsules comprising ramipril are sold in the United States and elsewhere under the tradename ALTACE™ in strengths of 1.25 mg. 2.5 mg, 5 mg and 10 mg. For all four strengths, the capsules are two-piece hard gelatin capsules filled with a mixture of ramipril and pregelatinized starch.
™ - trademark

Pregelatinized starch is thus the only excipient (i.e. inactive ingredient) with which the ramipril is mixed.

ACE inhibitors, such as ramipril, are generally very difficult to formulate into dosage forms because for most ACE inhibitors, contact with many of the excipients commonly used in pharmaceutical products accelerates the rate of degradation of the ACE inhibitor, so that the product is not sufficiently stable to enable long shelf-life. It is thus generally difficult to select the excipients that enable dosage forms with adequate stability.

For example, for the ACE inhibitor enalapril maleate, U.S. patent 5,562,921 discloses that stable tablets can be made comprising anhydrous lactose as filler and zinc stearate as lubricant.

WO 96/07400 relates to a pharmaceutical composition containing the ACE inhibitor ramipril and a dihydropyridine compound.

WO 00/34314 relates to pharmaceutical compositions comprising quinopril magnesium.

For certain other ACE inhibitors, and in particular quinapril, U.S. patent 4,830,853 disdoses that the compound can be stabilized against oxidation and discolourants by including ascorbic acid or sodium ascorbate in the composition; and U.S. patent 4,743,450 discloses that stability is improved by inclusion of an alkaline compound as stabilizer.

DE4420102 relates to a pharmaceutical preparation containing alpha-liponic acid and at least one organic nitrate, calcium antagonist, ACE inhibitor or oxyfedrin as its active ingredient.

EP0317878 relates to stabilised medicinal substances, a process for the preparation thereof and stable medicinal formulations.

For the ACE inhibitor fosinopril sodium, U.S. patent 5,006,344 teaches that compositions are relatively unstable if they comprise magnesium stearate as lubricant, but stability can be improved by use of sodium stearyl fumarate or hydrogenated vegetable oil as lubricant.

None of the aforesaid teachings appears to be of assistance in formulating stable solid compositions for oral administration (i.e. capsules or tablets) comprising ramipril.

As aforesaid, ramipril is disclosed in U.S. patent 5,061,722. With respect to the formulation of solid dosage forms for oral administration, the said patent teaches as follows:
"Examples of inert carriers which can be used are gum arabic, magnesium stearate, potassium phosphate, lactose, glucose and starch, especially starch."

WO 02/11709 relates to a stable,orally dosable pharmaceutical effervescent formulation comprising the ACE-inhibitor ramipril.

Also, as aforesaid, ALTACE™ capsules contain ramipril in admixture with pregelatinized starch as the sole diluent, presumably because the manufacturer found pregelatinized starch to be the diluent that enabled the best stability.

Although the stability of ALTACE™ capsules is sufficient to enable the capsules to be sold, the ramipril content does slowly degrade in ALTACE™ capsules, and it is desirable to enable solid dosage forms, and in particular capsules, with improved stability. The object of the present invention is thus to enable dosage forms comprising ramipril with stability superior to that obtained by diluting the ramipril with starch.

### SUMMARY OF THE INVENTION

It has surprisingly been found that, when lactose monohydrate is used as diluent, stability is superior to that achieved by using either anhydrous lactose or starch as diluent.

The invention is thus a solid pharmaceutical composition for oral administration comprising a mixture of ramipril with lactose monohydrate.

### DETAILED DESCRIPTION OF THE INVENTION

In the case of capsules comprising an active ingredient in amount of 25 mg or more per capsule, it is sometimes possible and practical to fill the capsules with pure active ingredient, without diluting the active ingredient with any excipient at all.

However, in the case of capsules comprising a smaller amount of active ingredient, it is generally necessary to dilute the active ingredient with one or more excipients and then to fill the mixture into the capsules.

Since ramipril capsules are sold in strengths of 1.25 mg, 2.5 mg, 5 mg and 10 mg, it is necessary to dilute the ramipril with one or more excipients.

There are many excipients that can be used as diluent in pharmaceutical capsules, including for example, starch, cellulose, calcium sulfate, calcium carbonate, dicalcium phosphate, lactose, dextrose, sucrose, dextrates, mannitol, maltodextrin, methylcellulose, and polyethylene glycol.

Depending on the excipient selected as the diluent, it may be necessary to include one or more other ingredients to serve, for example, as lubricant to avoid sticking to tooling, or as disintegrant to cause the contents of the capsules to disperse after the capsules is ingested and the shell is dissolved in gastric fluid. When starch is used as diluent (as done in ALTACE™), it is usually not necessary to include any other excipient, as starch has lubricant properties and disintegrant properties.

Lactose is available as both anhydrous lactose (with no water of hydration) and lactose monohydrate (with one mole of water of hydration per mole of lactose). As a general rule, anhydrous lactose, being free of water, would be expected to enable better stability than lactose monohydrate, particularly with ACE inhibitors, so it is particularly surprising that, in the case of ramipril, it has been found, as aforesaid, that lactose monohydrate as diluent enables better stability than use of either anhydrous lactose or starch.

As aforesaid, the invention is solid pharmaceutical compositions for oral administration comprising a mixture of ramipril with lactose monohydrate as diluent.

The composition may take the form of either a compressed tablet, or a two-piece hard gelatin capsule filled with a mixture comprising ramipril and lactose monohydrate.

The amount of ramipril per tablet or capsule will preferably be from 1.25 mg to 10 mg.

The amount of lactose monohydrate per tablet or capsule will preferably be from 25 mg to 200 mg and will more preferably be from 50 mg to 150 mg.

The composition will preferably further comprise another ingredient, which serves as a lubricant, to avoid sticking to tooling used to compress the tablet or fill the capsule.

The lubricant will preferably be a stearate such as magnesium stearate, zinc stearate or calcium stearate, and will more preferably be magnesium stearate. The amount of lubricant will preferably be from 0.2 mg to 2 mg per tablet or capsule, and will more preferably be from 0.5 mg to 1.5 mg per tablet or capsule.

The composition will also optionally comprise other excipients, such as, for example, starch, in admixture with the ramipril, lactose and lubricant.

The total amount of excipients other than lactose monohydrate will preferably be less than 50% of the composition by weight, more preferably less than 25%, even more preferably less than 10%, and most preferably less than 5%.

The invention will be further understood from the following examples.

| Examples: | 1 | 2 | 3 | 4 |
|---|---|---|---|---|
| Ramipril | 1.25 | 1.25 | 1.25 | 1.25 |
| Pregelatinized starch, undried | 148.75 | 0 | 0 | 0 |
| Pregelatinized starch, dried | 0 | 148.75 | 0 | 0 |
| Lactose anhydrous | 0 | 0 | 147.25 | 0 |
| Lactose monohydrate | 0 | 0 | 0 | 147.25 |
| Magnesium stearate | 0 | 0 | 1.5 | 1.5 |
| | 150 | 150 | 150 | 150 |

For each of the 4 examples, the ingredients in the proportions shown were mixed together. The powder mixture was then passed through a #60 screen and mixed again. The powder mixture was then filled into size 4 two-piece hard gelatin capsules as a net fill of 150 mg per capsules, so that each capsule contained 1.25 mg of ramipril.

Capsules of each of the examples were stored at 50°C for one week and then tested by a high-performance liquid chromatographic method (HPLC) to determine the degradation products as a percentage of the ramipril content.

The results were as follows:

| Example No. | Degradation Products |
|---|---|
| 1 | 2.58% |
| 2 | 2.93% |
| 3 | 3.11% |
| 4 | 1.10% |

The level of degradation products in the ramipril used to make the capsules was 0.29%. The increase in degradation products in the capsules of example 4 was thus only about 0.8% versus over 2% in each of the other three examples.

It is thus shown that the use of lactose monohydrate, as diluent, enables a lower degradation rate than the use of anhydrous lactose or starch (whether dried or undried).

## Claims

1. A solid pharmaceutical composition for oral administration comprising ramipril and lactose monohydrate, wherein the total amount of excipients other than lactose monohydrate is less than 50% of the composition by weight.

2. A composition of claim 1 wherein the total of excipients other than lactose monohydrate is less than 25% of the composition by weight.

3. A composition of claim 1 wherein the total amount of excipients other than lactose monohydrate is less than 10% of the composition by weight.

4. A composition of claim 1 wherein the total amount of excipients other than lactose monohydrate is less than 5% of the composition by weight.

5. A composition of claims 1 to 4 in the form of a tablet or the contents of a two-plece hard gelatin capsule, wherein the amount of ramipril per tablet or capsules is from 1.25 mg to 10 mg.

6. A composition of any of claims 1 to 5 wherein the amount of lactose monohydrate per tablet or capsule is from 25 mg to 200 mg.

7. A composition of claim 6 wherein the amount of lactose monohydrate per tablet or capsule is from 50 mg to 150 mg.

8. A composition of any of claims 1 to 7 further comprising a lubricant.

9. A composition of claim 8 wherein the lubricant is a stearate.

10. A composition of claim 9 wherein the lubricant is selected from the group consisting of magnesium stearate, zinc stearate and calcium stearate.

11. A composition of claim 10 wherein the lubricant is magnesium stearate.

12. A composition of any of claims 1 to 11 wherein the amount of lubricant per tablet or capsule is from 0.2 mg to 2 mg.

13. A composition of claim 12 wherein the amount of lubricant is from 0.5 mg to 1.5 mg.

## Patentansprüche

1. Feste pharmazeutische Zusammensetzung zur oralen Verabreichung, die Ramipril und Lactosemonohydrat aufweist, wobei die Gesamtmenge an Exzipientien, die anders sind als Lactosemonohydrat, weniger als 50 Gew.-% der Zusammensetzung beträgt.

2. Zusammensetzung nach Anspruch 1, wobei die Summe an Exzipientien, die anders sind als Lactosemonohydrat, weniger als 25 Gew.-% der Zusammensetzung beträgt.

3. Zusammensetzung nach Anspruch 1, wobei die Gesamtmenge an Exzipientien, die anders sind als Lactosemonohydrat, weniger als 10 Gew.-% der Zusammensetzung beträgt.

4. Zusammensetzung nach Anspruch 1, wobei die Gesamtmenge an Exzipientien, die anders sind als Lactosemonohydrat, weniger als 5 Gew.-% der Zusammensetzung beträgt.

5. Zusammensetzung nach den Ansprüchen 1 bis 4 in Form einer Tablette oder als Inhalt einer zweiteiligen Hartgelatinekapsel, wobei die Menge an Ramipril pro Tablette oder Kapsel 1,25 mg bis 10 mg beträgt.

6. Zusammensetzung nach einem der Ansprüche 1 bis 5, wobei die Menge an Lactosemonohydrat pro Tablette oder Kapsel 25 mg bis 200 mg beträgt.

7. Zusammensetzung nach Anspruch 6, wobei die Menge an Lactosemonohydrat pro Tablette oder Kapsel 50 mg bis 150 mg beträgt.

8. Zusammensetzung nach einem der Ansprüche 1 bis 7, die weiterhin ein Gleitmittel umfasst.

9. Zusammensetzung nach Anspruch 8, wobei das Gleitmittel ein Stearat ist.

10. Zusammensetzung nach Anspruch 9, wobei das Gleitmittel aus der Gruppe ausgewählt ist, bestehend aus Magnesiumstearat, Zinkstearat und Calciumstearat.

11. Zusammensetzung nach Anspruch 10, wobei das Gleitmittel Magnesiumstearat ist.

12. Zusammensetzung nach einem der Ansprüche 1 bis 11, wobei die Menge an Gleitmittel pro Tablette oder Kapsel 0,2 mg bis 2 mg beträgt.

13. Zusammensetzung nach Anspruch 12, wobei die Menge an Gleitmittel 0,5 mg bis 1,5 mg beträgt.

## Revendications

1. Composition pharmaceutique solide pour administration par voie orale, comprenant du ramipril et du monohydrate de lactose, dans laquelle la quantité totale d'excipients autres que le monohydrate de lactose est inférieure à 50 % de la composition en poids.

2. Composition selon la revendication 1, dans laquelle le total des excipients autres que le monohydrate de lactose est inférieur à 25 % de la composition en poids.

3. Composition selon la revendication 1, dans laquelle la quantité totale d'excipients autres que le monohydrate de lactose est inférieure à 10 % de la composition en poids.

4. Composition selon la revendication 1, dans laquelle la quantité totale d'excipients autres que le monohydrate de lactose est inférieure à 5 % de la composition en poids.

5. Composition selon les revendications 1 à 4 sous la forme d'un comprimé ou du contenu d'une capsule de gélatine dure en deux parties, dans laquelle la quantité de ramipril par comprimé ou capsule est de 1,25 mg à 10 mg.

6. Composition selon l'une quelconque des revendications 1 à 5, dans laquelle la quantité de monohydrate de lactose par comprimé ou capsule est de 25 mg à 200 mg.

7. Composition selon la revendication 6, dans laquelle la quantité de monohydrate de lactose par comprimé ou capsule est de 50 mg à 150 mg.

8. Composition selon l'une quelconque des revendications 1 à 7, comprenant en outre un lubrifiant.

9. Composition selon la revendication 8, dans laquelle le lubrifiant est un stéarate.

10. Composition selon la revendication 9, dans laquelle le lubrifiant est choisi dans le groupe constitué du stéarate de magnésium, du stéarate de zinc et du stéarate de calcium.

11. Composition selon la revendication 10, dans laquelle le lubrifiant est le stéarate de magnésium.

12. Composition selon l'une quelconque des revendications 1 à 11, dans laquelle la quantité de lubrifiant par comprimé ou capsule est de 0,2 mg à 2 mg.

13. Composition selon la revendication 12, dans laquelle la quantité de lubrifiant est de 0,5 mg à 1,5 mg.
